# EUROPEAN PATENT APPLICATION

(11) **EP 4 635 437 A1**
(43) Date of publication of application: **22.10.2025**
(21) Application number: 23903353.3
(22) Date of filing: 05.12.2023
(51) Int. Cl.: A61B 18/12

(54) **ABLATION SYSTEM AND OPERATING METHOD THEREFOR**

(30) Priority: 12.12.2022 JP 2022198054
(71) Applicant: KANEKA CORPORATION, Osaka 530-8288 (JP)
(72) Inventor: KISA, Toshiya, Okaya-shi, Nagano 394-0034 (JP)
(74) Representative: Hoffmann Eitle
(86) International application number: PCT/JP2023/043408
(87) International publication number: WO 2024/128064

(57) **Abstract**

Provided is an ablation system that make it possible to observe the myocardium in real time during ablation while reducing noise in the ultrasound images caused by the application of voltage. An ablation system (10) having an insertion member (20) having a distal end and a proximal end and extending in a longitudinal direction; at least one ablation electrode (30) disposed at a distal part of the insertion member (20); a transducer (40) disposed at a distal part of the insertion member (20) and capable of transmitting and receiving ultrasound; and a pulse voltage generator (50) electrically connected to the ablation electrode (30).

## Description

### TECHNICAL FIELD

The present invention relates to an ablation system and a method for operating the same.

### BACKGROUND ART

Conventionally, in order to treat arrhythmias such as atrial fibrillation, a procedure called pulmonary vein isolation has been performed, in which tissue at the inlet of a pulmonary vein within the heart is ablated using so-called ablation techniques. In such a procedure, an ablation catheter is used, in which a high-frequency current is supplied to electrodes provided at a distal end of a catheter while the electrodes are in contact with the inlet of the pulmonary vein, thereby ablating the tissue of the pulmonary vein inlet. During ablation, ultrasound may be used to monitor the degree of myocardial ablation. For example, Patent document 1 discloses a medical device including a catheter having a ring-shaped distal end, electrodes disposed on the distal end, and a shaft extending proximally from the distal end, wherein an ultrasonic transducer fixed to the distal end of the shaft images a region near the distal end based on signals detected by the ultrasonic transducer. Patent documents 2 and 3 also disclose ablation systems including image sensors provided with image acquisition elements or ultrasonic transducers. Patent document 4 discloses a system including a catheter provided with electrodes that perform functions such as ablation, and an ultrasonic imaging sensor. In this system, electrical signals based on ultrasonic echoes are transmitted from the catheter to an image processor, which displays ultrasonic images in real time.

### RELATED ART DOCUMENTS

### PATENT DOCUMENTS

Patent document 1: JP-A-2012-130702
Patent document 2: JP-A-2003-524499
Patent document 3: JP-A-2019-515756
Patent document 4: JP-A-2007-044507

### SUMMARY OF THE INVENTION

### PROBLEMS TO BE SOLVED BY THE INVENTION

However, in the above conventional ablation system, it has sometimes been difficult to acquire ultrasonic images due to noise caused by the application of voltage for supplying a high-frequency current to the electrode.

In view of the above circumstances, the present invention is directed to providing an ablation system and a method for operating the same, which make it possible to observe the myocardium in real time during ablation while reducing noise in the ultrasonic images caused by the application of voltage.

### MEANS FOR SOLVING THE PROBLEMS

An ablation system according to an embodiment of the present invention, which is capable of addressing the above-described issues, is as follows.
[1] An ablation system, comprising: an insertion member having a distal end and a proximal end and extending in a longitudinal direction; at least one ablation electrode disposed at a distal part of the insertion member; a transducer disposed at a distal part of the insertion member and capable of transmitting and receiving ultrasound; and a pulse voltage generator electrically connected to the ablation electrode.
   The ablation system according to an embodiment of the present invention is preferably any one of the following [2] to [9].
[2] The ablation system according to [1], further comprising: a signal generator electrically connected to the transducer; and a controller configured to cause the signal generator to generate a signal to the transducer in timing between pulse voltages generated by the pulse voltage generator.
[3] The ablation system according to [2], wherein the controller is configured to cause the transducer to transmit and receive signals in synchronization with an ECG signal obtained by electrocardiographic measurement.
[4] The ablation system according to any one of [1] to [3], wherein the insertion member has a tip member at a distal part thereof, and the ablation electrode is disposed on the tip member.
[5] The ablation system according to any one of [1] to [4], wherein the transducer includes a plurality of ultrasound transducer elements.
[6] The ablation system according to [5], wherein the ultrasound transducer elements include a first ultrasound transducer element group and a second ultrasound transducer element group, the first ultrasound transducer element group is disposed distal to the ablation electrode, and the second ultrasound transducer element group is disposed proximal to the ablation electrode.
[7] The ablation system according to any one of [1] to [6], wherein the insertion member has a lumen extending in the longitudinal direction, the insertion member has a shaft that is slidably disposed in the lumen and that has a distal end and a proximal end, and the transducer is disposed at a distal part of the shaft.
[8] The ablation system according to [7], wherein the shaft is rotatable about its axis.
[9] The ablation system according to any one of [1] to [8], further comprising an ultrasound imaging device electrically connected to the transducer.
   A method for operating the ablation system according to an embodiment of the present invention is as follows.
[10] A method for operating the ablation system according to any one of above [1] to [9], comprising: a step of supplying pulse currents to the ablation electrode by the pulse voltage generator; and a step of receiving or transmitting ultrasound by the transducer in timing between the pulse currents.
   The method for operating the ablation system according to an embodiment of the present invention is preferably any one of the following [11] to [13].
[11] The method for operating the ablation system according to [10], wherein the ablation system further comprises a signal generator electrically connected to the transducer and a controller configured to cause the signal generator to generate a signal to the transducer in timing between the pulse currents generated by the pulse voltage generator, wherein the controller is configured to cause the transducer to transmit and receive signals in synchronization with an ECG signal obtained by electrocardiographic measurement.
[12] The method for operating the ablation system according to [10] or [11], wherein the ablation system further comprises an ultrasound imaging device electrically connected to the transducer; the insertion member has a lumen extending in the longitudinal direction, the insertion member has a shaft that is slidably disposed in the lumen and that has a distal end and a proximal end; the transducer is disposed at a distal part of the shaft; and the ultrasound imaging device is configured to image signals from the transducer while the shaft is rotated.
[13] The method for operating the ablation system according to [12], wherein the transducer includes a plurality of ultrasound transducer elements, and the ultrasound transducer elements include a first ultrasound transducer element group and a second ultrasound transducer element group; the first ultrasound transducer element group is disposed distal to the ablation electrode, and the second ultrasound transducer element group is disposed proximal to the ablation electrode; and the ultrasound imaging device is configured to image signals from the first ultrasound transducer element group and the second ultrasound transducer element group.

### EFFECTS OF THE INVENTION

According to the above-described ablation system and the method for operating the same, it is possible to observe the myocardium in real time during ablation while reducing noise in the ultrasound images caused by the application of voltage. As a result, appropriate ablation can be performed while monitoring the condition of the ablated myocardium in real time.

### BRIEF DESCRIPTION OF THE DRAWINGS

[FIG. 1] FIG. 1 is a side view of an ablation system according to an embodiment of the present invention.
[FIG. 2] FIG. 2 is a perspective view of a distal part of the ablation system shown in FIG. 1.
[FIG. 3] FIG. 3 is a graph showing the relationship between a pulse voltage generated by the pulse voltage generator and time.
[FIG. 4] FIG. 4 is a graph showing a modified example of FIG. 3.
[FIG. 5] FIG. 5 is a graph showing another modified example of FIG. 3.
[FIG. 6] FIG. 6 is a graph showing still another modified example of FIG. 3.
[FIG. 7] FIG. 7 is a schematic view of image information obtained by the ablation system shown in FIG. 1.
[FIG. 8] FIG. 8 is a side view of an ablation system according to another embodiment of the present invention.
[FIG. 9] FIG. 9 is a side view of a distal portion of an ablation system according to yet another embodiment of the present invention.
[FIG. 10] FIG. 10 is a perspective view showing a modified example of FIG. 2.

### MODE FOR CARRYING OUT THE INVENTION

Hereinafter, the present invention will be described based on the following embodiments, however, the present invention is not limited by the following embodiments and can be altered in design within a scope in compliance with the intent described above and below, and all the changes are to be encompassed within a technical scope of the present invention. Note that, in each drawing, hatching, reference signs for components, and the like may be omitted for convenience of description, and in such a case, the specification and other drawings are to be referred to. Furthermore, since the dimensions of the various components in the drawings are provided for the purpose of facilitating the understanding of the feature of the present invention, the dimensions may differ from the actual dimensions in some cases.

### 1. Ablation system

An ablation system according to an embodiment of the present invention has an insertion member having a distal end and a proximal end and extending in a longitudinal direction; at least one ablation electrode disposed at a distal part of the insertion member; a transducer disposed at a distal part of the insertion member and capable of transmitting and receiving ultrasound; and a pulse voltage generator electrically connected to the ablation electrode.

The ablation system includes a catheter, and one example of its application is pulmonary vein isolation, which is a treatment for atrial fibrillation. In pulmonary vein isolation, an ablation electrode is provided on the outer surface of a distal part of the catheter, and the pulmonary vein inlet is ablated by supplying a high-frequency current to the ablation electrode while the electrode is in contact with the pulmonary vein inlet. This allows abnormal electrical pathways that cause atrial fibrillation to be isolated.

In this case, it is possible to carry out ablation treatment while checking the condition of the myocardium by obtaining image information of the myocardium using ultrasound during ablation. However, due to noise caused by the application of voltage for supplying a high-frequency current, it may be difficult to obtain sufficient image information to observe the depth of myocardial ablation. The ablation system having the above configuration includes at least one ablation electrode and a transducer capable of receiving ultrasound, both of which are disposed at a distal part of an insertion member, and a pulse voltage generator is electrically connected to the ablation electrode. As a result, it is possible to observe the myocardium in real time during ablation while reducing noise in the image information caused by the application of current. This enables appropriate ablation to be performed while monitoring the condition of the ablated myocardium in real time.

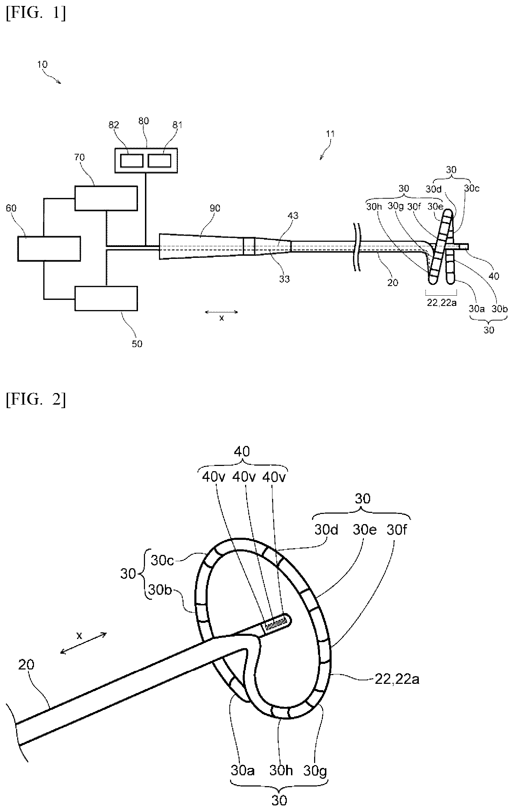

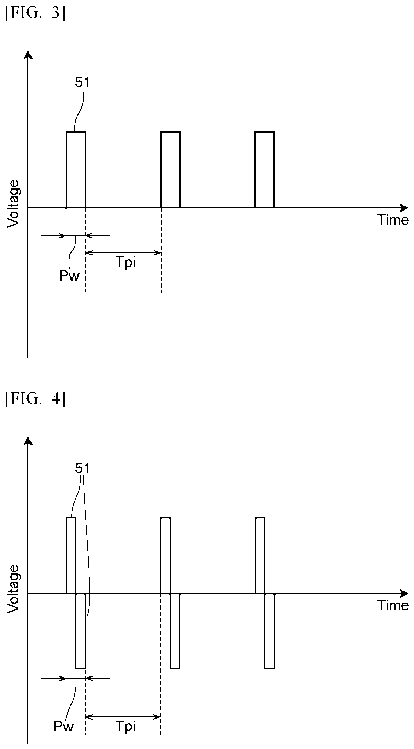

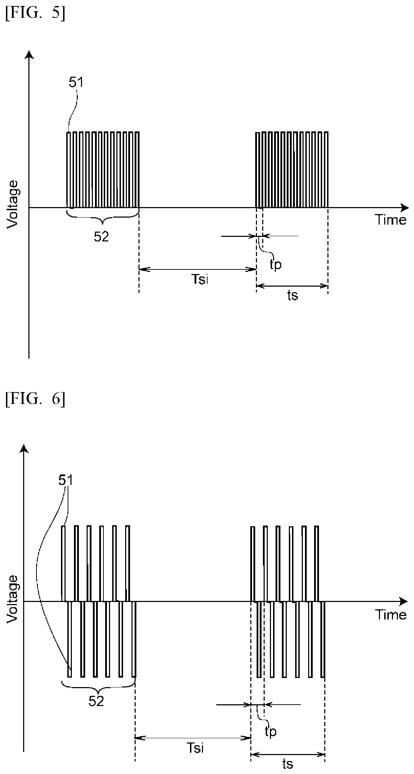

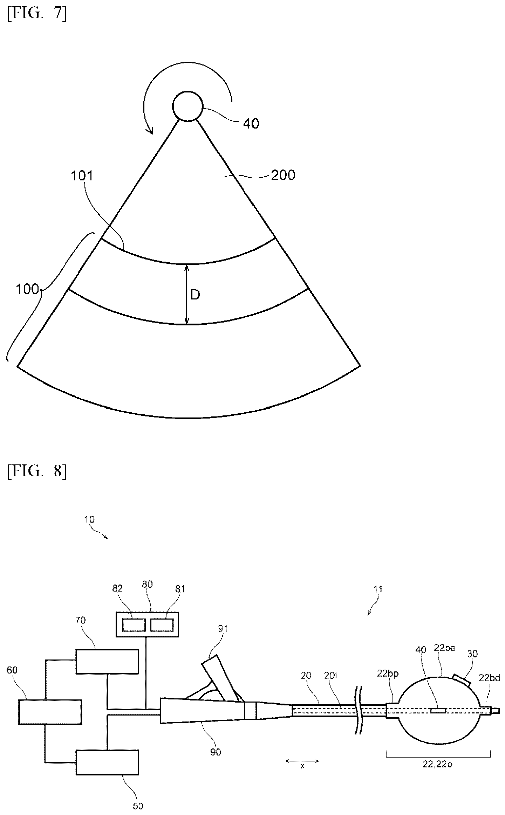

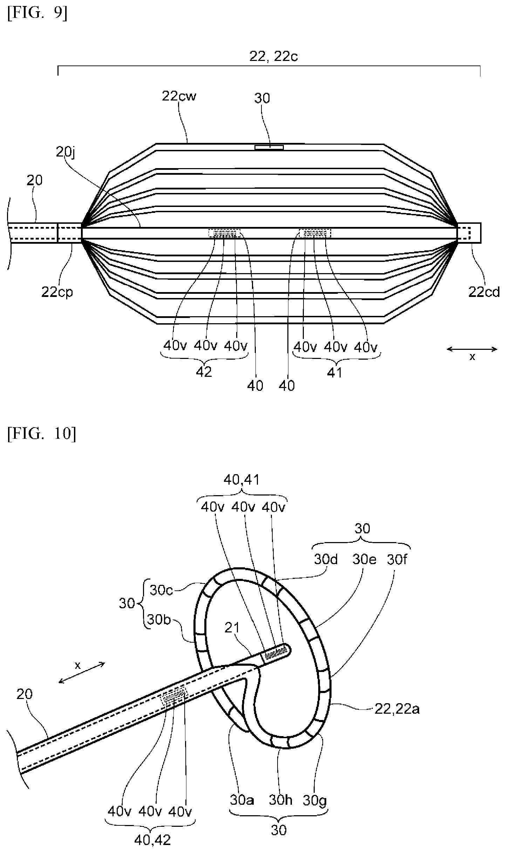

Hereinafter, an ablation system according to an embodiment of the present invention will be described with reference to FIGs. 1 to 10. FIG. 1 is a side view of an ablation system according to an embodiment of the present invention, illustrating an embodiment in which an insertion member has a ring as a tip member. FIG. 2 is a perspective view of a distal part of the ablation system shown in FIG. 1. FIG. 3 is a graph showing the relationship between a pulse voltage generated by the pulse voltage generator and time, and FIGs. 4 to 6 are graphs showing modified examples of FIG. 3. In FIGs. 3 to 6, the generated pulse voltages are schematically illustrated, and the numbers of pulse voltages shown in FIGs. 5 and 6 do not necessarily represent the actual number of pulse voltages constituting one pulse train. FIG. 7 is a schematic view of image information obtained by the ablation system shown in FIG. 1. FIG. 8 is a side view of an ablation system according to another embodiment of the present invention, illustrating an embodiment in which the insertion member has a balloon as a tip member. FIG. 9 is a side view of a distal part of an ablation system according to yet another embodiment of the present invention, illustrating an embodiment in which the insertion member has a basket as a tip member. FIG. 10 is a perspective view showing a modified example of FIG. 2.

As shown in FIG. 1, an ablation system 10 according to an embodiment of the present invention includes: an insertion member 20 that has a distal end and a proximal end and extends in a longitudinal direction x; at least one ablation electrode 30 disposed at a distal part of the insertion member 20; a transducer 40 capable of transmitting and receiving ultrasound, also disposed at a distal part of the insertion member 20; and a pulse voltage generator 50 electrically connected to the ablation electrode 30. In the present specification, the portion including the insertion member 20, the ablation electrode 30, and the transducer 40 may be referred to as a catheter 11. That is, the ablation system 10 has a configuration including the catheter 11 and the pulse voltage generator 50.

The insertion member 20 has a longitudinal direction x, a radial direction defined in a cross section perpendicular to the longitudinal direction x as a direction connecting a centroid of an outer edge of the insertion member 20 and a point on the outer edge, and a circumferential direction along the outer edge in the cross section perpendicular to the longitudinal direction x.

In the present specification, the longitudinal direction x of the insertion member 20 may simply be referred to as the longitudinal direction x when describing the ablation system 10. The distal side of the catheter 11 refers to the side toward the subject to be treated in the longitudinal direction x, while the proximal side refers to the opposite side, that is, the side closer to the user's hand.

As shown in FIG. 1, the insertion member 20 has a distal end and a proximal end and extends in the longitudinal direction x, and is a member that is inserted into the body from the distal end side. Preferably, the catheter 11 includes a handle 90 on the proximal side of the insertion member 20. The proximal side of the catheter 11 including the handle 90 is positioned outside the body, and the catheter 11 can be operated by gripping the handle 90.

Although not shown in the figures, the insertion member 20 may have a configuration in which multiple elongated members are joined together in the longitudinal direction x. In this case, the respective outer diameters of the elongated members may be different from one another, and at least some of the elongated members may be tubes, with an end of one elongated member being disposed within a lumen of another tube.

Preferably, the insertion member 20 is flexible so that it can deform along the shape of a body cavity when inserted into the body, and preferably has elasticity. The insertion member 20 is preferably made of resin, metal, or a combination of resin and metal. Examples of the insertion member 20 include: an elongated resin member or a resin tube; an elongated metal member or a metal tube, or one having resin coating on at least one of the inner or outer surfaces thereof; an elongated member or hollow body formed by arranging wires in a predetermined pattern, or one having resin coating on at least one of the inner or outer surfaces thereof; or combinations thereof, such as those connected in the longitudinal direction x. The elongated resin member or resin tube can be manufactured, for example, by extrusion molding. Examples of the hollow body in which wires are arranged in a predetermined pattern include a tubular body having a mesh structure formed by intersecting or braiding wires, and a coil formed by winding wires. The wires may be one or more single wires or one or more stranded wires. When the insertion member 20 is made of metal, cuts or grooves may be formed on the outer surface of the insertion member 20 to enhance its flexibility. The shapes of the cuts or grooves may be linear, arcuate, annular, spiral, or a combination thereof.

Examples of resins constituting the insertion member 20 include synthetic resins such as polyolefin-based resins such as polyethylene and polypropylene; polyamide-based resins such as nylon; polyester-based resins such as PET; aromatic polyether ketone-based resins such as PEEK; polyether-polyamide-based resins; polyurethane-based resins; polyimide-based resins; fluorine-based resins such as PTFE, PFA, and ETFE; and polyvinyl chloride-based resins. These resins may be used alone or in combination of two or more. Examples of metals constituting the insertion member 20 include stainless steels such as SUS304 and SUS316; carbon steel; metals such as platinum, nickel, cobalt, chromium, titanium, tungsten, and gold; and alloys such as Ni-Ti alloys and Co-Cr alloys. These may also be used alone or in combination of two or more.

The outer diameter of the insertion member 20 is preferably 1 mm or more, and more preferably 2 mm or more. This makes it possible to ensure the rigidity of the insertion member 20. The outer diameter of the insertion member 20 is preferably 5 mm or less, and more preferably 4 mm or less. This makes it possible to facilitate the insertion of the insertion member 20 into the body.

At least one ablation electrode 30 is disposed on the outside of the distal part of the insertion member 20. The ablation electrode 30 can generate heat by the flow of electric current and ablate a target tissue. FIG. 1 shows an embodiment in which a plurality of ablation electrodes 30 (30a to 30h) are disposed, but only one ablation electrode 30 may be disposed. The number of ablation electrodes 30 to be disposed is not particularly limited when a plurality of them are provided, but may be, for example, two or more, three or more, four or more, or twenty or less, fifteen or less, or ten or less. By disposing a plurality of ablation electrodes 30, it becomes easy to ablate a wide area at once.

Although not shown in the figures, when only one ablation electrode 30 is disposed, a voltage may be applied between the ablation electrode 30 and a return electrode provided on the body surface of the patient, and the ablation electrode 30 may be used as a monopolar electrode. Alternatively, a plurality of ablation electrodes 30 may be provided, and a voltage may be applied between one of these ablation electrodes 30 and the return electrode.

Alternatively, a voltage may be applied between a plurality of ablation electrodes 30, and the ablation electrodes 30 may be used as bipolar electrodes. Since electric current can be passed locally as compared with the monopolar configuration, it is possible to prevent ablation of tissues other than the target tissue.

As the ablation electrode 30, a thin film or sheet of a metal oxide or metal can be used. This makes it easy to provide the ablation electrode 30 on the surface of the insertion member 20. Methods for forming a thin-film ablation electrode 30 include etching, vacuum deposition, sputtering, ion plating, plating, coating, and printing methods such as screen printing and offset printing.

The material constituting the ablation electrode 30 only needs to have conductivity, and may be composed of, for example, a metal or a mixture containing a metal and a resin. Among them, preferably, a conductive resin or a metal such as gold, silver, copper, platinum, platinum-iridium alloy, stainless steel, or tungsten is used. The materials constituting each of the plurality of ablation electrodes 30 may be the same or different from each other. Preferably, the materials are the same from the viewpoint of facilitating the control of the electric current flowing through the ablation electrodes 30.

FIGs. 1 and 2 show a cylindrical ablation electrode 30 that covers the surface of the insertion member 20, but the shape of the ablation electrode 30 is not limited thereto, and may be any shape such as a circular shape, an elliptical shape, a polygonal shape, a prismatic shape, or a combination thereof. When a plurality of ablation electrodes 30 are provided, the thickness of each of the plurality of ablation electrodes 30 may be the same or different from each other, preferably being the same. When the thickness of each of the plurality of ablation electrodes 30 is the same, it becomes easier to bring each ablation electrode 30 into contact with the target tissue to the same degree, making it easier to control the degree of ablation.

The ablation electrode 30 is connected to a first conductive wire 33, and the first conductive wire 33 preferably extends to the proximal side and is connected to a pulse voltage generator 50, which will be described later. When a high-frequency pulse voltage generated by the pulse voltage generator 50 is applied, the ablation electrode 30 is heated, making it possible to ablate the tissue that is in contact with the ablation electrode 30. Alternatively, the ablation electrode 30 may be used not only for ablation of tissue but also for measurement of bioelectric potential. The bioelectric potential can be obtained, for example, by measuring the potential difference between a reference electrode preferably provided on the catheter 11 and the ablation electrode 30. As the reference electrode, an electrode arranged separately from the ablation electrode 30 or an electrode attached to the body surface of the patient can be used.

The first conductive wire 33 may be a conductive linear body such as a conductive wire, a conductive substance printed on an insulating material, or a connection of these. The first conductive wire 33 can be disposed on the outer surface of the insertion member 20 or, when the insertion member 20 has a lumen, within the lumen.

As shown in FIG. 1, a transducer 40 capable of transmitting and receiving ultrasound is disposed at a distal part of the insertion member 20. FIG. 1 shows an embodiment in which the transducer 40 is provided at the distal end part of the insertion member 20, but the position at which the transducer 40 is disposed is not particularly limited as long as it is near the ablation electrode 30, and the transducer 40 may be provided distal to the ablation electrode 30, proximal to the ablation electrode 30, or at a position overlapping the ablation electrode 30 in the longitudinal direction x.

Preferably, the transducer 40 has an ultrasound transducer element that converts an electrical signal into ultrasound and emits the ultrasound, and also converts received ultrasound into an electrical signal. By irradiating a target tissue with ultrasound emitted from the transducer 40 and receiving the reflected ultrasound with the transducer 40, it is possible to obtain information on the target tissue, specifically, the information about the depth direction of the wall of the myocardium, which is a specific example of the target tissue.

The transducer 40 may be of a single-transducer type or of a dual-transducer type having a transmitting transducer and a receiving transducer. The transducer can have a configuration including two electrodes and a piezoelectric material sandwiched between the two electrodes. The piezoelectric material is a crystalline substance exhibiting piezoelectricity, and is a material that generates a voltage when mechanical strain is applied, or conversely, generates mechanical strain when a voltage is applied. As the piezoelectric material, barium titanate, lead zirconate titanate, polyvinylidene fluoride, or 1-3 composite piezoelectric materials can be used.

As shown in FIG. 1, preferably, the transducer 40 is connected to a signal generator 70, which will be described later, via a second conductive wire 43. The method of arrangement and the material of the second conductive wire 43 can be referred to the above description regarding the method of arrangement and the material of the first conductive wire 33.

The ablation system 10 has a pulse voltage generator 50 that is electrically connected to the ablation electrode 30. As schematically shown in FIGs. 3 and 4, the pulse voltage generator 50 can generate a pulse voltage 51. The pulse voltage generator 50 may generate a monophasic pulse, that is, a direct current pulse, as schematically shown in FIG. 3, or may generate a biphasic pulse, as schematically shown in FIG. 4.

In the embodiment of the present invention, preferably, the pulse voltage 51 is a rectangular voltage pulse. The duration during which the pulse voltage 51 is applied, that is, the pulse width Pw, is preferably 100 nanoseconds or more, more preferably 300 nanoseconds or more, and even more preferably 500 nanoseconds or more, and is preferably 100 microseconds or less, more preferably 50 microseconds or less, and even more preferably 10 microseconds or less. The maximum amplitude of the pulse voltage 51 is preferably 500 V or more, more preferably 700 V or more, and even more preferably 1000 V or more, and is preferably 3000 V or less, more preferably 2500 V or less, and even more preferably 2000 V or less. The interval Tpi between the pulse voltages 51 is preferably 100 nanoseconds or more, more preferably 300 nanoseconds or more, and even more preferably 500 nanoseconds or more, and may be 1 microsecond or more, and is preferably 10 milliseconds or less, more preferably 5 milliseconds or less, even more preferably 1 millisecond or less, and may also be 500 microseconds or less, 100 microseconds or less, 50 microseconds or less, or 10 microseconds or less. By repeatedly applying the pulse voltage 51 at the interval Tpi, the ablation electrode 30 can be heated to ablate the target tissue. The time required for a single ablation by repeated application of the pulse voltage 51 is preferably 1 millisecond or more, more preferably 5 milliseconds or more, and even more preferably 10 milliseconds or more, and is preferably 500 milliseconds or less, more preferably 300 milliseconds or less, and even more preferably 100 milliseconds or less.

Unlike conventional ablation catheters in which a high-frequency voltage is applied to the ablation electrode 30 for several seconds, in the ablation system 10, the pulse voltage 51 in the order of nanoseconds to microseconds is applied, and the interval Tpi between the pulse voltages 51 is in the order of nanoseconds to milliseconds, so that transmission and reception of ultrasound can be performed by the transducer 40 during a period in which no voltage is applied between the pulse voltages 51. This makes it possible to reduce the influence of noise caused by the application of high-frequency voltage during ultrasound transmission and reception. That is, the timing of ablation by voltage application and the timing of observation of the myocardium by ultrasound transmission and reception can be shifted, thereby making it possible to obtain ultrasound image information with a high S/N ratio. Here, the timing shift depends on the pulse width Pw and the interval Tpi between the pulse voltages 51, and since the duration is in the order of nanoseconds to milliseconds, it can be said that, from the perspective of the time required for one ablation, the ablation and the observation of the myocardium are performed simultaneously, making it possible to perform treatment while appropriately advancing the ablation by monitoring the state of the ablated myocardium in real time.

In addition, by applying the pulse voltage 51 to the ablation electrode 30 using the pulse voltage generator 50, the duration during which current flows through the ablation electrode 30 can be shortened. Therefore, unlike conventional ablation catheters in which high-frequency voltage is applied for several seconds, it is possible to reduce the disadvantage of excessive temperature rise in the myocardium and to prevent accidents such as esophageal perforation.

As schematically shown in FIGs. 5 and 6, the pulse voltage generator 50 may generate the pulse voltage 51 as a pulse train 52. That is, the pulse voltage generator 50 may repeatedly apply the pulse train 52 at an interval Tsi. The pulse train 52 can include a plurality of pulse voltages 51 at a period tp within a time ts (length of the pulse train), and can be repeatedly generated at the interval Tsi. The period tp, which is the time during which one pulse voltage 51 is generated within the pulse train 52, is preferably 0.2 microseconds or more, more preferably 0.5 microseconds or more, and even more preferably 1 microsecond or more, and is preferably 20 microseconds or less, more preferably 15 microseconds or less, and even more preferably 10 microseconds or less. The length ts of one pulse train is preferably 0.05 milliseconds or more, more preferably 0.1 milliseconds or more, and even more preferably 0.5 milliseconds or more, and may be 1 millisecond or more or 5 milliseconds or more, and is preferably 100 milliseconds or less, more preferably 50 milliseconds or less, and even more preferably 10 milliseconds or less. The interval Tsi between pulse trains 52 is preferably 0.05 microseconds or more, more preferably 0.1 microseconds or more, and even more preferably 0.5 microseconds or more, and may be 1 microsecond or more or 5 microseconds or more, and is preferably 100 milliseconds or less, more preferably 50 milliseconds or less, even more preferably 10 milliseconds or less, and may also be 1 millisecond or less, 0.5 milliseconds or less, 0.1 milliseconds or less, or 0.05 milliseconds or less.

The pulse voltage generator 50 may generate a monophasic pulse train 52 as schematically shown in FIG. 5, or may generate a biphasic pulse train 52 as schematically shown in FIG. 6. In either case, the pulse voltage generator 50 can repeatedly generate the pulse train 52, which includes a plurality of pulse voltages 51, at an interval Tsi.

By generating the pulse voltage 51 as a pulse train 52, the pulse voltage generator 50 can improve ablation efficiency. Then, by repeatedly generating the pulse train 52 at the interval Tsi, transmission and reception of ultrasound can be performed by the transducer 40 during the interval Tsi. This makes it possible to improve ablation efficiency through the application of the pulse train 52 while shifting the timing between ablation by voltage application and observation of the state of the myocardium by ultrasound transmission and reception, thereby enabling acquisition of ultrasound image information with a high S/N ratio. Here, the timing shift depends on the length ts of the pulse train 52 and the interval Tsi, and since the length is in the order of microseconds to milliseconds, it can be said that ablation and observation of the myocardium are performed simultaneously, making it possible to perform treatment efficiently while appropriately advancing ablation by monitoring the state of the ablated myocardium in real time.

Even in the case where the pulse train 52 is applied, the duration during which current flows through the ablation electrode 30 can be shortened, thereby reducing the disadvantage of excessive temperature rise in the myocardium and making it possible to prevent accidents such as esophageal perforation.

Preferably, the application of the pulse train 52 by the pulse voltage generator 50 and the transmission and reception of ultrasound by the transducer 40 are alternately repeated at high speed while shifting their timing. In one ablation, the number of times the pulse train 52 is applied is preferably 3 times or more, more preferably 5 times or more, and even more preferably 10 times or more, and is preferably 1000 times or less, more preferably 500 times or less, and even more preferably 100 times or less. Preferably, the transmission and reception of ultrasound are also performed the same number of times as described above during the interval of the length Tsi between the pulse trains 52.

As shown in FIG. 1, preferably, the ablation system 10 has an ultrasound imaging device 80 that is electrically connected to the transducer 40. Furthermore, the ultrasound imaging device 80 preferably has an image generation unit 81 and an image display unit 82. The ultrasound received by the transducer 40 includes ultrasound reflected from the myocardium, and preferably, the image generation unit 81 generates image information based on the electrical signal converted from this ultrasound, and displays an image of the myocardium on the image display unit 82. As a result, it is possible to obtain an ultrasound image as shown in FIG. 7, and for example, when ablation is performed while observing the pulmonary vein inlet of the left atrium, it is possible to obtain image information of the surface 101 of the myocardium, which is the boundary between the myocardium 100 and the blood 200 in the left atrium, and the depth D of the ablated region.

As shown in FIG. 1, preferably, the ablation system 10 further has a signal generator 70 that is electrically connected to the transducer 40, and a controller 60 that causes the signal generator 70 to transmit a signal to the transducer 40 at the timing of the interval Tpi, that is, between the pulse voltage 51 and the pulse voltage 51 generated by the pulse voltage generator 50. Alternatively, the controller 60 may cause the signal generator 70 to transmit a signal to the transducer 40 at the timing of the length Tsi between the pulse train 52 and the pulse train 52 generated by the pulse voltage generator 50. This makes it easier to perform, during the interval Tpi between the pulse voltages 51 or the interval Tsi between the pulse trains 52, the process in which the transducer 40, having received a signal from the signal generator 70, irradiates a target tissue with ultrasound and obtains image information from the reflected ultrasound, and makes it easier to obtain ultrasound images at the timing of the interval Tpi between the pulse voltages 51 or the interval Tsi between the pulse trains 52.

Preferably, the signal generator 70 has a transmission processing section that causes the transducer 40 to generate ultrasound in accordance with a transmission signal output from the controller 60. Furthermore, preferably, the signal generator 70 has a bias processing section that applies a DC bias to the transducer 40 in accordance with the transmission signal output from the controller 60. The bias processing section may include a DC voltage source, a resistor, a decoupling capacitor, or the like.

The signal generator 70 may also have a reception processing section that converts ultrasound reflected from a target tissue by the transducer 40 into an electrical signal and outputs it to the ultrasound imaging device 80. Furthermore, preferably, the signal generator 70 has a switch section that switches, at a predetermined timing, between the supply of the transmission signal from the controller 60 to the transmission processing section and the output of the electrical signal from the reception processing section to the ultrasound imaging device 80.

Preferably, the controller 60 causes the transducer 40 to transmit and receive signals in synchronization with an ECG signal obtained through electrocardiographic measurement. The ECG signal includes a P wave, a QRS wave, and a T wave, and by causing the transducer 40 to transmit and receive signals at a timing other than the P wave, which corresponds to atrial contraction, and the QRS wave, which corresponds to ventricular contraction, it is possible to reduce the influence of heart contraction on ultrasound images. Alternatively, the transmission and reception of signals by the transducer 40 may be performed at any of the timings of the P wave, the QRS wave, and the T wave, but for example, the system may be configured so that the transmission and reception of signals by the transducer 40 is performed at a predetermined time after a specific wave (e.g., 10 milliseconds to 100 milliseconds later). As a result, it is possible to obtain ultrasound images in which the heart is always in the same state of contraction or expansion, thereby reducing the influence of cardiac motion on the ultrasound images. Consequently, since the influence of both noise caused by the current flowing through the ablation electrode 30 and the contraction and expansion of the heart is reduced, it becomes possible to more accurately understand the ablation state of the myocardium.

As shown in FIGs. 1, 2, 8, and 9, preferably, the insertion member 20 has a tip member 22 at a distal part thereof, and the ablation electrode 30 is disposed on the tip member 22. The shape of the tip member 22 is not particularly limited, and examples include a ring 22a as shown in FIGs. 1 and 2, a balloon 22b as shown in FIG. 8, and a basket 22c as shown in FIG. 9.

With reference to FIGs. 1 and 2, a configuration in which the insertion member 20 has a ring 22a as the tip member 22 will be described. Preferably, the ring 22a is formed in an arc shape or a spiral shape around the central axis of the insertion member 20. The ring 22a may be formed in a shape composed of a plurality of arcs, that is, in a coil shape. The ring 22a may be an integral part formed with the insertion member 20 as a single member, or may be joined to the insertion member 20 by a joining method such as adhesion with an adhesive, welding, or swaging of a ring-shaped member.

Preferably, the ablation electrode 30 is disposed on the outer surface of the ring 22a. With such a configuration, it is possible to ablate the myocardium over a wide area in the radial direction of the insertion member 20 by means of the ablation electrode 30. **In** this case, preferably, a plurality of ablation electrodes 30 are provided on the surface of the ring 22a. Preferably, the plurality of ablation electrodes 30 are provided at approximately equal intervals on the surface of the ring 22a, as shown by the ablation electrodes 30a to 30h in FIGs. 1 and 2. This makes it easier to ablate a wide area of the myocardium.

The first conductive wire 33 connected to the ablation electrode 30 provided on the surface of the ring 22a may be disposed outside the insertion member 20 and connected to the pulse voltage generator 50, or may extend within a lumen of the insertion member 20, if the insertion member 20 has a lumen, and be connected to the pulse voltage generator 50.

The material constituting the ring 22a can be referred to the above description regarding the material constituting the insertion member 20.

When the insertion member 20 has the ring 22a as the tip member 22, preferably, the transducer 40 is not provided on the ring 22a. This makes it easier to perform ablation by the ablation electrode 30 provided on the ring 22a and acquisition of ultrasound images by the transducer 40. In this case, the transducer 40 may be provided distal to the ring 22a, proximal to the ring 22a, or at a position overlapping the ring 22a in the longitudinal direction x.

The second conductive wire 43 connected to the transducer 40 may be disposed outside the insertion member 20 and connected to the signal generator 70 and/or the ultrasound imaging device 80, or may extend within a lumen of the insertion member 20, if the insertion member 20 has a lumen, and be connected to the signal generator 70 and/or the ultrasound imaging device 80.

With reference to FIG. 8, a configuration in which the insertion member 20 has a balloon 22b as the tip member 22 will be described. Preferably, the balloon 22b has an inflation part 22be, a distal fixing part 22bd disposed distal to the inflation part 22be, and a proximal fixing part 22bp disposed proximal to the inflation part 22be. Preferably, the insertion member 20 has a lumen extending in the longitudinal direction x, and has an inner tube 20i disposed in the lumen. This makes it possible to extend the inner tube 20i distally from the lumen of the insertion member 20 and adopt a configuration in which the distal fixing part 22bd of the balloon 22b is fixed to the distal end part of the inner tube 20i, and the proximal fixing part 22bp of the balloon 22b is fixed to the insertion member 20. As methods for fixing the balloon 22b, there may be used adhesion with an adhesive, welding, or swaging of a ring-shaped member.

The material constituting the inner tube 20i can be referred to the above description regarding the material constituting the insertion member 20.

The catheter 11 may have a so-called over-the-wire type configuration in which the inner tube 20i extends to the proximal end part of the insertion member 20 and functions as a guidewire lumen into which a guidewire is inserted from a guidewire port provided on a handle 90. Alternatively, the catheter 11 may have a so-called rapid exchange type configuration in which the inner tube 20i extends to a guidewire port provided partway to the proximal end of the insertion member 20 and functions as a guidewire lumen.

Preferably, the balloon 22b has a configuration in which the inflation part 22be inflates by introducing a fluid into an internal lumen. The fluid can be introduced into the internal lumen of the balloon 22b through a passage that is inside the insertion member 20 and outside the inner tube 20i. When the catheter 11 has an over-the-wire type configuration, the catheter 11 has a fluid inlet 91 branched from the handle 90, and the fluid may be introduced from the fluid inlet 91.

The balloon 22b can be manufactured by molding a resin. For example, the balloon 22b can be manufactured by placing an extruded resin tube in a mold and forming it by blow molding. Instead of blow molding, molding methods such as dip molding, injection molding, or compression molding may also be used. Examples of resins constituting the balloon 22b include polyamide-based resins, polyester-based resins, polyurethane-based resins, polyolefin-based resins, vinyl chloride-based resins, silicone-based resins, natural rubber, and synthetic rubber. These may be used singly or in combination of two or more. From the viewpoint of thinning and flexibility of the balloon 22b, it is preferable to use elastomer resins.

Preferably, the ablation electrode 30 is disposed on the outer surface of the balloon 22b. This makes it easier to bring the ablation electrode 30 into contact with the myocardium by inflating the balloon 22b. One ablation electrode 30 may be provided, or a plurality of ablation electrodes 30 may be arranged on the balloon 22b in the longitudinal direction x and/or in the circumferential direction. Although not shown in the figures, preferably, the plurality of ablation electrodes 30 are arranged at approximately equal intervals in the longitudinal direction x or the circumferential direction of the balloon 22b. This makes it easier to ablate a wide area at once by inflating the balloon 22b.

The first conductive wire 33 connected to the ablation electrode 30 provided on the surface of the balloon 22b may be disposed outside the insertion member 20 from the outer surface of the balloon 22b and connected to the pulse voltage generator 50, or may extend within a lumen of the insertion member 20, if the insertion member 20 has a lumen, and be connected to the pulse voltage generator 50. Alternatively, the insertion member 20 may have another layer on its outside, and the first conductive wire 33 may extend outside the insertion member 20 and inside the other layer.

Preferably, the transducer 40 is disposed on the inner tube 20i arranged inside the balloon 22b; although it may be disposed outside the inner tube 20i, more preferably, it is disposed inside the inner tube 20i. This allows the transducer 40 to be protected by the inner tube 20i. Preferably, the second conductive wire 43 connected to the transducer 40 extends within the lumen of the inner tube 20i and is connected to the signal generator 70 and/or the ultrasound imaging device 80. Alternatively, the transducer 40 may be provided on the balloon 22b. Although the first conductive wire 33 and the second conductive wire 43 in the configuration in which the insertion member 20 has the balloon 22b as the tip member 22 are not shown in the figures, reference can be made to FIG. 1, which shows a configuration in which the insertion member 20 has the ring 22a as the tip member 22.

With reference to FIG. 9, a configuration in which the insertion member 20 has a basket 22c as the tip member 22 will be described. Preferably, the basket 22c has a plurality of wires 22cw extending in the longitudinal direction x, and the distal end parts and proximal end parts of the plurality of wires 22cw are bound together. The basket 22c may be configured such that it remains in a contracted state while disposed within the lumen of a tubular body such as a guiding catheter, and expands when released outside the lumen. Although not shown in the figures, the basket 22c may further have wires that are separate from the wires 22cw extending in the longitudinal direction x, wherein the wires are connected to the longitudinal wires 22cw and extend in the circumferential direction. That is, the plurality of wires 22cw may be connected in a mesh structure.

Preferably, the basket 22c has a distal binding part 22cd at which the distal end parts of the plurality of wires 22cw are bound, and a proximal binding part 22cp at which the plurality of wires 22cw are bound on the proximal side relative to the distal binding part 22cd. With such a configuration, the basket 22c can expand more easily. Preferably, at the distal binding part 22cd and the proximal binding part 22cp, the plurality of wires 22cw are bound by a method such as thermal welding, silver brazing, adhesion, or swaging.

As shown in FIG. 9, preferably, the insertion member 20 has a lumen extending in the longitudinal direction x and has an inner tube 20j disposed in the lumen. This makes it possible to extend the inner tube 20j distally from the lumen of the insertion member 20 and form the basket 22c by a plurality of wires 22cw using the inner tube 20j as an axis.

The material constituting the inner tube 20j can be referred to the above description regarding the material constituting the insertion member 20.

The plurality of wires 22cw may or may not contain a conductive material. Preferably, the plurality of wires 22cw are made of a shape-memory alloy or a shape-memory resin, and may be made, for example, of stainless steel such as SUS304 or SUS316; metals such as platinum, nickel, cobalt, chromium, titanium, tungsten, aluminum, gold, or silver; or alloys such as Ni-Ti alloys or Co-Cr alloys. The number of the plurality of wires 22cw is not particularly limited, and may be, for example, 3 or more, 4 or more, 5 or more, or 10 or more, and 20 or less or 15 or less.

Preferably, the ablation electrode 30 is disposed on the outer surface of the basket 22c, that is, on the outer surfaces of the plurality of wires 22cw constituting the basket 22c. This makes it easier to bring the ablation electrode 30 into contact with the myocardium by expanding the basket 22c. One ablation electrode 30 may be provided. Alternatively, the catheter 11 may have a configuration in which a plurality of ablation electrodes 30 are provided along the longitudinal direction x of a single wire 22cw, a configuration in which one ablation electrode 30 is provided on each of a plurality of wires 22cw so that the basket 22c as a whole has a plurality of ablation electrodes 30, or a configuration including a combination thereof. Although not shown in the figures, preferably, the plurality of ablation electrodes 30 are arranged at approximately equal intervals in the longitudinal direction x or in the circumferential direction of the basket 22c. This makes it easier to ablate a wide area at once by expanding the basket 22c.

The first conductive wire 33 connected to the ablation electrode 30 provided on the surface of the basket 22c may be disposed outside the insertion member 20 from the surface of the wire 22cw and connected to the pulse voltage generator 50, or may extend within a lumen of the insertion member 20, if the insertion member 20 has a lumen, and be connected to the pulse voltage generator 50. Alternatively, the insertion member 20 may have another layer on its outside, and the first conductive wire 33 may extend outside the insertion member 20 and inside the other layer.

Preferably, the transducer 40 is disposed on the inner tube 20j serving as the axis of the basket 22c; although it may be disposed outside the inner tube 20j, more preferably, it is disposed inside. This allows the transducer 40 to be protected by the inner tube 20j. Preferably, the second conductive wire 43 connected to the transducer 40 extends within the lumen of the inner tube 20j and is connected to the signal generator 70 and/or the ultrasound imaging device 80. Alternatively, the transducer 40 may be provided on the wire 22cw constituting the basket 22c. Although the first conductive wire 33 and the second conductive wire 43 in the configuration in which the insertion member 20 has the basket 22c as the tip member 22 are not shown in the figures, reference can be made to FIG. 1, which shows a configuration in which the insertion member 20 has the ring 22a as the tip member 22.

The transducer 40 may have one ultrasound transducer element 40v, but preferably, as shown in FIG. 2, it has a plurality of ultrasound transducer elements 40v. Preferably, each of the plurality of ultrasound transducer elements 40v is arranged so as to be adjacent to each other in the longitudinal direction x. With such an array-type transducer, the transducer 40 can receive ultrasound over a wide area. In addition, this enables the transducer 40 to transmit ultrasound over a wide area. Although not shown in the figures, the transducer 40 may have a plurality of ultrasound transducer elements 40v arranged adjacent to each other in the circumferential direction of the insertion member 20. Furthermore, the transducer 40 may have both a plurality of ultrasound transducer elements 40v arranged so as to be adjacent to each other in the longitudinal direction x and a plurality of ultrasound transducer elements 40v arranged so as to be adjacent to each other in the circumferential direction of the insertion member 20. Preferably, the number of ultrasound transducer elements 40v is 1 or more, 2 or more, 3 or more, 4 or more, 6 or more, 8 or more, 12 or more, 20 or more, 24 or more, 32 or more, 64 or more, and 120 or less, 100 or less, or 90 or less.

When the transducer 40 has a plurality of ultrasound transducer elements 40v, it may be configured to emit ultrasound with timing shifted for each ultrasound transducer element 40v. This makes it possible to generate a focused beam by synthesizing the wavefronts of the multiple ultrasonic waves. Furthermore, the transducer 40 may be configured to receive reflected ultrasound with timing shifted for each ultrasound transducer element 40v. Even in such a case where the timing is shifted for each ultrasound transducer element 40v, preferably, the transmission and reception of ultrasound by the transducer 40 are performed during the interval Tpi between the pulse voltages 51 generated by the pulse voltage generator 50, or during the interval Tsi between the pulse trains 52. This makes it possible to obtain an ultrasound image while reducing noise caused by the voltage generation by the pulse voltage generator 50.

Preferably, the ultrasound transducer element 40v has a protrusion. Preferably, the protrusion projects outward, and more preferably, it projects toward the target to which ultrasound is transmitted. Preferably, the shape of the protrusion is a quadrangular pyramid frustum, a prism, a cylinder, or a conical frustum, and more preferably, a quadrangular pyramid frustum or a prism.

As shown in FIGs. 9 and 10, preferably, the ultrasound transducer elements 40v include a first ultrasound transducer element group 41 and a second ultrasound transducer element group 42. Preferably, the first ultrasound transducer element group 41 is disposed distal to the ablation electrode 30, and the second ultrasound transducer element group 42 is disposed proximal to the ablation electrode 30. Preferably, the first ultrasound transducer element group 41 and the second ultrasound transducer element group 42 are each arranged adjacent to one another in the longitudinal direction x. Alternatively, each of the first ultrasound transducer element group 41 and the second ultrasound transducer element group 42 may have a plurality of ultrasound transducer elements 40v arranged adjacent to each other in the circumferential direction of the insertion member 20. Furthermore, each of the first ultrasound transducer element group 41 and the second ultrasound transducer element group 42 may have both a plurality of ultrasound transducer elements 40v arranged adjacent to each other in the longitudinal direction x and a plurality of ultrasound transducer elements 40v arranged adjacent to each other in the circumferential direction of the insertion member 20. Preferably, the number of ultrasound transducer elements 40v in each of the first ultrasound transducer element group 41 and the second ultrasound transducer element group 42 is 1 or more, 2 or more, 3 or more, 4 or more, 6 or more, 8 or more, 12 or more, 20 or more, 24 or more, 32 or more, 64 or more, and 120 or less, 100 or less, or 90 or less.

By disposing the first ultrasound transducer element group 41 and the second ultrasound transducer element group 42 on the distal side and the proximal side of the ablation electrode 30, respectively, in the longitudinal direction x, it becomes possible to make the resulting ultrasound image more three-dimensional and to obtain more detailed information regarding the ablation state of the myocardium by the ablation electrode 30.

As shown in FIG. 10, preferably, the insertion member 20 has a lumen extending in the longitudinal direction x, and has a shaft 21 that has a distal end and a proximal end and is slidably disposed in the lumen. The transducer 40 is preferably disposed on the distal part of the shaft 21. With such a configuration, the transducer 40 can be easily positioned within the insertion member 20 while taking into account the position relative to the ablation electrode 30. Furthermore, since the shaft 21 is slidable within the lumen of the insertion member 20, when the catheter 11 is delivered into a body cavity, the shaft 21 can be accommodated within the lumen of the insertion member 20 to improve the insertability of the insertion member 20 into the body cavity, and when performing ultrasonic observation, the distal part of the shaft 21 can be exposed from the lumen of the insertion member 20 as necessary.

Although not shown in the figures, regardless of what type of member is used as the tip member 22 of the insertion member 20, the insertion member 20 may have another lumen in addition to the lumen in which the shaft 21 is disposed.

As shown in FIG. 10, it is also preferable that the transducer 40 including the first ultrasound transducer element group 41 is disposed at the distal end part of the shaft 21, and that the transducer 40 including the second ultrasound transducer element group 42 is disposed on the shaft 21 at a position proximal to the transducer 40 including the first ultrasound transducer element group 41. In such a configuration, it is also possible to perform ultrasonic observation by exposing only the transducer 40 including the first ultrasound transducer element group 41 from the lumen of the insertion member 20, while keeping the transducer 40 including the second ultrasound transducer element group 42 disposed within the lumen of the insertion member 20.

Preferably, the shaft 21 is rotatable about its axis. This allows the transducer 40 disposed at the distal part of the shaft 21 to be rotated about the axis as indicated by the arrows in FIG. 7, thereby making it possible to obtain ultrasound images over a wide angular range. If the shaft 21 is rotated 360 degrees, it is also possible to obtain ultrasound images over a full 360-degree range.

### 2. Method for operating ablation system

The present invention also provides a method for operating the ablation system 10. Hereinafter, the method for operating the ablation system 10 may simply be referred to as the "operation method." The configuration of the ablation system 10 can be referred to in the above section "1. Ablation system."

The operation method according to the embodiment of the present invention includes a step of supplying pulse currents to the ablation electrode 30 by the pulse voltage generator 50; and a step of receiving or transmitting ultrasound by the transducer 40 in timing between the pulse currents. By allowing the transducer 40 to receive or transmit ultrasound at the timing between the pulse currents, it is possible to observe the condition of the myocardium in real time while performing ablation with the ablation electrode 30, while reducing noise in the image information obtained by ultrasound transmission and reception due to current application. As a result, it becomes possible to perform appropriate ablation while monitoring the state of the ablated myocardium in real time.

As schematically shown in FIGs. 3 and 4, the pulse voltage generator 50 can supply a pulse current to the ablation electrode 30 by repeatedly generating a pulse voltage 51 at the interval Tpi. **In** this case, the pulse voltage generator 50 may generate a monophasic pulse, i.e., a direct current pulse as shown in FIG. 3, or may generate a biphasic pulse as shown in FIG. 4. Alternatively, as schematically shown in FIGs. 5 and 6, the pulse voltage generator 50 may generate the pulse voltage 51 as a pulse train 52, and a pulse current train based on the pulse train 52 may be repeatedly supplied at the interval Tsi. As a result, since the transmitting and receiving member 40 can transmit and receive ultrasound during the intervals Tpi and Tsi in which no current flows to the ablation electrode 30, it becomes possible to obtain an ultrasound image with reduced noise caused by the current. Consequently, it becomes possible to perform treatment by appropriately proceeding with the ablation while monitoring the condition of the ablated myocardium in real time.

The ablation system 10 preferably further includes a signal generator 70 electrically connected to the transducer 40, and a controller 60 that causes the signal generator 70 to generate a signal to the transducer 40 in timing between the pulse currents supplied by the pulse voltage generator 50. In the operation method according to the embodiment of the present invention, preferably, the controller 60 causes the transducer 40 to transmit and receive signals in synchronization with an ECG signal obtained through electrocardiogram measurement. At this time, by causing the transducer 40 to transmit and receive signals at a timing other than the P wave in which the atrium contracts or the QRS wave in which the ventricle contracts, it is possible to reduce the influence of heart contraction on the ultrasound image. Alternatively, the signal transmission and reception by the transducer 40 may be performed at the timing of any of the P wave, QRS wave, or T wave. For example, it may be configured such that the transducer 40 transmits and receives signals a predetermined time (e.g., 10 milliseconds to 100 milliseconds) after a specific wave. As a result, since ultrasound images can be obtained with the heart in a constant state of contraction or expansion, the influence of cardiac motion on the ultrasound image can be reduced. Consequently, it becomes easier to accurately grasp the ablation condition of the myocardium, since the ultrasound image is less affected by both the noise due to the high-frequency current flowing through the ablation electrode 30 and the contraction and expansion of the heart.

Preferably, the ablation system 10 further includes an ultrasound imaging device 80 electrically connected to the transducer 40; the insertion member 20 has a lumen extending in the longitudinal direction x, and includes a shaft 21 that has a distal end and a proximal end and is slidably disposed in the lumen; and the transducer 40 is disposed at a distal part of the shaft 21. In the operation method according to the embodiment of the present invention, it is preferable that the ultrasound imaging device 80 images signals from the transducer 40 while the shaft 21 is rotated. As a result, since the transducer 40 disposed at the distal part of the shaft 21 can be rotationally moved about the axis as indicated by the arrows in FIG. 7, it becomes possible to obtain ultrasound images over a wide angular range. By rotating the shaft 21 through 360 degrees, it is also possible to obtain ultrasound images over a full 360-degree range.

Preferably, the transducer 40 includes a plurality of ultrasound transducer elements 40v, which include a first ultrasound transducer element group 41 and a second ultrasound transducer element group 42; and the first ultrasound transducer element group 41 is disposed on the distal side relative to the ablation electrode 30, and the second ultrasound transducer element group 42 is disposed on the proximal side relative to the ablation electrode 30. In the operation method according to the embodiment of the present invention, it is preferable that the ultrasound imaging device 80 images signals from both the first ultrasound transducer element group 41 and the second ultrasound transducer element group 42. As a result, the obtained ultrasound images can be made more three-dimensional, enabling more detailed information to be obtained regarding the ablation state of the myocardium by the ablation electrode 30.

The present application claims priority based on Japanese Patent Application No. 2022-198054 filed on December 12, 2022. All the contents described in Japanese Patent Application No. 2022-198054 filed on December 12, 2022 are incorporated herein by reference.

### DESCRIPTION OF REFERENCE SIGNS

10: ablation system
11: catheter
20: insertion member
20i, 20j: inner tube
21: shaft
22: tip member
22a: ring
22b: balloon
22bd: distal fixing part
22be: inflation part
22bp: proximal fixing part
22c: basket
22cd: distal binding part
22cp: proximal binding part
22cw: wire
30, 30a - 30h: ablation electrode
33: first conductive wire
40: transducer
40v: ultrasound transducer element
41: first ultrasound transducer element group
42: second ultrasound transducer element group
43: second conductive wire
50: pulse voltage generator
51: pulse voltage
52: pulse train
60: controller
70: signal generator
80: ultrasound imaging device
81: image generation unit
82: image display unit
90: handle
91: fluid inlet
100: myocardium
101: surface of the myocardium
200: blood in the left atrium
D: depth of ablation
Pw: pulse width
tp: period of pulse
ts: length of pulse train
Tpi: interval between pulse voltages
Tsi: interval between pulse train

## Claims

1. An ablation system, comprising:
an insertion member having a distal end and a proximal end and extending in a longitudinal direction;
at least one ablation electrode disposed at a distal part of the insertion member;
a transducer disposed at a distal part of the insertion member and capable of transmitting and receiving ultrasound; and
a pulse voltage generator electrically connected to the ablation electrode.

2. The ablation system according to claim 1, further comprising:
a signal generator electrically connected to the transducer; and
a controller configured to cause the signal generator to generate a signal to the transducer in timing between pulse voltages generated by the pulse voltage generator.

3. The ablation system according to claim 2, wherein the controller is configured to cause the transducer to transmit and receive signals in synchronization with an ECG signal obtained by electrocardiographic measurement.

4. The ablation system according to claim 1 or 2, wherein the insertion member has a tip member at a distal part thereof, and the ablation electrode is disposed on the tip member.

5. The ablation system according to claim 1 or 2, wherein the transducer includes a plurality of ultrasound transducer elements.

6. The ablation system according to claim 5, wherein the ultrasound transducer elements include a first ultrasound transducer element group and a second ultrasound transducer element group, the first ultrasound transducer element group is disposed distal to the ablation electrode, and the second ultrasound transducer element group is disposed proximal to the ablation electrode.

7. The ablation system according to claim 1 or 2, wherein the insertion member has a lumen extending in the longitudinal direction, the insertion member has a shaft that is slidably disposed in the lumen and that has a distal end and a proximal end, and the transducer is disposed at a distal part of the shaft.

8. The ablation system according to claim 7, wherein the shaft is rotatable about its axis.

9. The ablation system according to claim 1 or 2, further comprising an ultrasound imaging device electrically connected to the transducer.

10. A method for operating the ablation system according to claim 1, comprising:
a step of supplying pulse currents to the ablation electrode by the pulse voltage generator; and
a step of receiving or transmitting ultrasound by the transducer in timing between the pulse currents.

11. The method for operating the ablation system according to claim 10, wherein
the ablation system further comprises a signal generator electrically connected to the transducer and a controller configured to cause the signal generator to generate a signal to the transducer in timing between the pulse currents generated by the pulse voltage generator, wherein
the controller is configured to cause the transducer to transmit and receive signals in synchronization with an ECG signal obtained by electrocardiographic measurement.

12. The method for operating the ablation system according to claim 10 or 11, wherein
the ablation system further comprises an ultrasound imaging device electrically connected to the transducer;
the insertion member has a lumen extending in the longitudinal direction, the insertion member has a shaft that is slidably disposed in the lumen and that has a distal end and a proximal end;
the transducer is disposed at a distal part of the shaft; and
the ultrasound imaging device is configured to image signals from the transducer while the shaft is rotated.

13. The method for operating the ablation system according to claim 12, wherein
the transducer includes a plurality of ultrasound transducer elements, and the ultrasound transducer elements include a first ultrasound transducer element group and a second ultrasound transducer element group;
the first ultrasound transducer element group is disposed distal to the ablation electrode, and the second ultrasound transducer element group is disposed proximal to the ablation electrode; and
the ultrasound imaging device is configured to image signals from the first ultrasound transducer element group and the second group of ultrasound transducer elements.
